# EUROPÄISCHE PATENTSCHRIFT

(11) **EP 2 974 689 B1**
(45) Veröffentlichungstag und Bekanntmachung des Hinweises auf die Patenterteilung: **23.05.2018**
(21) Anmeldenummer: 15002103.8
(22) Anmeldetag: 15.07.2015
(51) Int. Cl.: A61C 13/00, A61C 13/12, F27B 17/02, F27D 5/00, A61C 13/20, B22F 3/10

(54) **ANORDNUNG ZUM SINTERN EINES DENTALWERKSTÜCKS**
ASSEMBLY FOR SINTERING A DENTAL WORK PIECE
SYSTEME DE FRITTAGE D'UNE PIECE DENTAIRE

(30) Priorität: 18.07.2014 AT 5692014
(43) Veröffentlichungstag der Anmeldung: 20.01.2016
(73) Patentinhaber: STEGER, Heinrich, 39031 Bruneck (IT)
(72) Erfinder: STEGER, Heinrich, 39031 Bruneck (IT)
(74) Vertreter: Torggler & Hofinger Patentanwälte

(56) Entgegenhaltungen:
- EP-A1- 1 712 534
- US-A- 4 299 567

## Beschreibung

Die Erfindung betrifft eine Anordnung zum Sintern eines Dentalwerkstücks, mit einem bearbeiteten Dentalwerkstück und einer Haltevorrichtung für das Dentalwerkstück. Zudem betrifft die Erfindung ein Verfahren zum Sintern eines Dentalwerkstücks.

Bei der Herstellung von Dentalwerkstücken, z. B. aus Sintermetallteilen, besteht eine große Herausforderung in der Sinterung der Grünlinge. Bei komplexeren Arbeiten, besonders im Dentalbereich, ist es von großer Wichtigkeit, dass es beim Sintern nicht zu einem unerwünschten Verzug der Arbeiten kommt. So war es bis jetzt nur möglich,
- kleinere Brückenarbeiten ohne problematischen Verzug herzustellen.

Aus dem Stand der Technik sind auch bereits diverse Möglichkeiten bekannt, um den Sintervorgang zu verbessern, jedoch weisen sämtliche bekannte Methoden Nachteile auf.

Beispielsweise zeigt die EP 1 154 969 B1 ein Verfahren zum dimensionstreuen Sintern von keramischen Formgegenständen. Ein solcher keramischer Formgegenstand kann z. B. ein Zahnersatzteil sein. In dieser Schrift ist angeführt, dass das Brenngut über Haken aufgehängt wird, wobei diese Haken aus feuerfestem Material, beispielsweise Aluminiumoxid, bestehen. Die Aufhängung kann auch über beweglich angebrachte Haken erfolgen. Weiters ist angeführt, dass Haltestifte aus dem gleichen Material wie das Brenngut bestehen. Z. B. sind die Haltestifte aus dem gleichen Rohling gefertigt. Nachteilig bei diesem Verfahren ist, dass das Aufhängen über zumindest zwei Haken erfolgen muss, was einen größeren Material- und Herstellungsaufwand bedeutet. Weiters muss immer der Schwerpunkt und die genaue Lage des Dentalwerkstücks eingestellt werden. Zudem berühren die Haken immer direkt das Dentalwerkstück bzw. den Formgegenstand, was beim Sintern ungewünschte Abdrücke hinterlassen kann.

Die EP 1 868 961 B1 wie auch die EP 1 712 534 A1 zeigen eine Einrichtung für dimensionsstabiles Sintern von vorgeformten keramischen Dentalartikeln, aufweisend ein Aufhängsystem. Dieses Aufhängsystem weist eine U-förmige Schaukel auf. Auch hierbei ist nachteilig, dass das Aufhängen immer über mindestens zwei Aufhängelemente erfolgt. Auch hier ist nachteilig, dass diese schaukeiförmigen Aufhängelemente direkt am keramischen Dentalartikel anliegen.

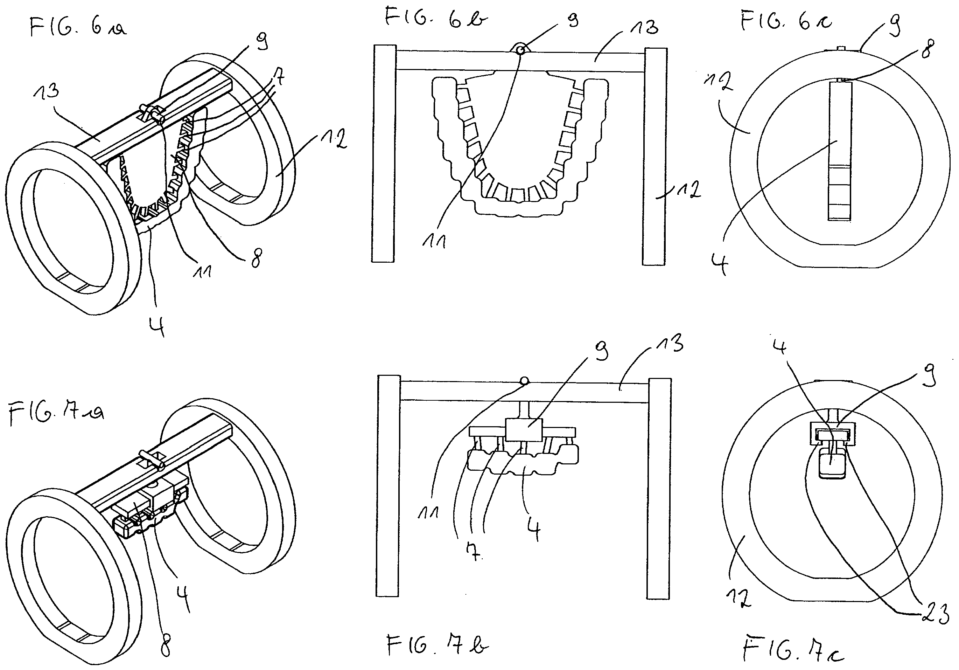

Weiters kann noch auf die EP 2 014 254 A1 verwiesen werden, welche eine zirkuläre Brücke (bridge structure) zeigt, die mit einem Halteteil (support body) über Verbindungselemente (retaining sections) verbunden ist. Z. B. aus Fig. 6 dieser Schrift geht hervor, dass das Halteteil, die Verbindungselemente und die Brückenkonstruktion einstückig ausgeführt sind. Eine derartige Konstruktion ist allerdings nur bei kleinen, wie in Fig. 6 dargestellt, dreigliedrigen Dentalwerkstücken sinnvoll, wobei auch bereits bei diesen recht kleinen Dentalwerkstücken sehr viel an Material für das das Dentalwerkstück umgebende Halteteil verwendet werden muss. Dies bringt einen erheblichen Materialverbrauch und ist sehr aufwendig. Aus diesem Grund ist in der EP 2 014 254 A1 auch ein weiteres Ausführungsbeispiel (siehe Fig. 12 bis 18) dargelegt, bei welchem eine recht große vielgliedrige zirkuläre Brücke dargestellt ist. Diese Brücke wird an einem stehenden Halteteil in Form eines Sinterfußes angebracht. Dieser Sinterfuß ist über kleine Stege mit den einzelnen Zähnen verbunden. Der Stand- bzw. Sinterfuß dient vornehmlich dazu, dass die Zähne nicht mit der Brennkammer in Kontakt kommen. Wenn nun diese auf dem Standfuß stehende Brücke gesintert wird, kann es zu einem großen Verzug kommen; In den seitlichen Bereichen ist dieser Verzug noch relativ gering, da dort die Schwerkraft gleichmäßig auf alle Glieder wirkt. Im oberen Bereich kann es beim Sintern allerdings dazu kommen, dass die einzelnen Glieder aufgrund der dünnen Verbindungselemente nach hinten oder vorne wegkippen, sodass ein ungewünschter Verzug entsteht. Das heißt, werden die Verbindungselemente auf Druck beansprucht, kommt es zu einer Verkürzung der beim Sintern weich werdenden Verbindungselemente und somit fast unweigerlich zur besagten Verkippung bzw. zu einer ungewünschten Verbiegung.

Dieselben Nachteile wie bei der letztgenannten Schrift sind auch bei der DE 10 2009 044 461 A1 gegeben, welche ein Verfahren zum Dimensionstreuen Sintern eines Formteils zeigt.

Aus der US 4,299,567 geht die Herstellung einer Dentalprothese hervor, wobei die Dentalrestauration auf separaten Stiften steht, welche wiederum von einer Basis gehalten werden.

Die Aufgabe der vorliegenden Erfindung besteht daher darin, eine gegenüber dem Stand der Technik verbesserte Anordnung bzw. ein verbessertes Verfahren zu schaffen. Insbesondere soll beim Sintern möglichst kein Verzug auftreten, der Materialbedarf gering sein und die Oberfläche des Dentalwerkstücks nicht beeinträchtigt werden.
Dies wird durch eine Anordnung mit den Merkmalen von Anspruch 1 erreicht. Demnach ist vorgesehen, dass das Dentalwerkstück eine Dentalkonstruktion mit zumindest fünf, vorzugsweise zumindest acht, miteinander verbundenen Gliedern aufweist (vorzugsweise entspricht dabei jedes Glied zumindest bereichsweise einem Zahnersatz eines einzelnen menschlichen Zahnes), das Dentalwerkstück an der Haltevorrichtung aufgehängt ist und das Dentalwerkstück über ein im oder am Dentalwerkstück ausgebildetes, von der Dentalkonstruktion gesondertes Verbindungsstück mit der Haltevorrichtung verbunden bzw. verbindbar ist. Dadurch ist eine Möglichkeit geschaffen, um auch große Dentalwerkstücke ohne Verzug, mit wenig Materialverbrauch und ohne ungewünschte Abdrücke herzustellen. Der Verzug wird vor allem dadurch unterbunden, dass das Dentalwerkstück an der Haltevorrichtung aufgehängt ist. Die Abdrücke auf dem Dentalwerkstück werden vor allem durch das von der Dentalkonstruktion gesonderte Verbindungsstück vermieden. Ein großer Materialverschleiß wird dadurch vermieden, dass bei großen, zumindest fünfgliedrigen Dentalkonstruktionen keine aufwendigen zusätzlichen, mit dem Dentalwerkstück einstückigen Haltekomponenten um die Glieder des Dentalwerkstücks herum vorgesehen sein müssen, sondern nur teilweise auf einer Seite des Dentalwerkstücks angeordnet sind. Durch das Aufhängen des Dentalwerkstücks kann zusätzlich erreicht werden, dass die Arbeit gleichmäßiger durch Wärmestrahlung erreicht wird.
Die Dentalkonstruktion kann prinzipiell recht lange gerade Abschnitte aufweisen, wobei die Dentalkonstruktion zumindest fünf Glieder aufweist. Jedes Glied entspricht dabei - zumindest bereichsweise - einem Zahnersatz eines einzelnen menschlichen Zahnes. Die einzelnen Glieder der Dentalkonstruktion müssen dabei nicht jeweils einen kompletten Zahn und somit auch nicht die Dentalkonstruktion einem kompletten Gebiss entsprechen. Vielmehr können einzelne Bereiche bzw. Glieder ja auch so ausgebildet sein, dass auf diesen später aufgebaut wird und dadurch die komplette Zahnform jedes einzelnen Gliedes entsteht. Dies ist beispielsweise bei einem sogenannten Steg der Fall. Deshalb müssen also diese Glieder nur Bereiche eines späteren Zahnersatzes entsprechen.

Bevorzugt ist allerdings vorgesehen, dass die Dentalkonstruktion zumindest bereichsweise zirkulär ausgebildet ist. Das heißt, die Dentalkonstruktion weist bereits eine Biegung bzw. Krümmung auf, die einer natürlich Krümmung bzw. Biegung im menschlichen Gebiss entspricht. Wenn die Dentalkonstruktion zumindest acht Glieder aufweist, ist diese Krümmung oder Biegung noch deutlicher erkennbar. Besondere Vorteile bringt die vorliegende Erfindung dann, wenn die Dentalkonstruktion vollständig zirkulär ausgebildet ist. Vollständig zirkulär bedeutet, dass die Dentalkonstruktion zumindest 12- bis 16-gliedrig ausgebildet ist. Mit anderen Worten ist somit bevorzugt vorgesehen, dass die Dentalkonstruktion als zumindest bereichsweise, vorzugsweise vollständig, zirkulär geformte Brückenkonstruktion oder als zumindest bereichsweise, vorzugsweise vollständig, zirkulär geformter Steg ausgebildet ist.

Für eine konstruktiv einfache Ausbildung mit wenig Materialverbrauch ist bevorzugt vorgesehen, dass das Dentalwerkstück Verbindungsstege und ein Basiselement aufweist, wobei die Dentalkonstruktion über die Verbindungsstege mit dem Basiselement verbunden ist. Um die Dentalkonstruktion beim Aufhängen nicht zu verformen, ist besonders bevorzugt vorgesehen, dass die Aufhängung über das Basiselement erfolgt. Im Speziellen kann dies dadurch erreicht werden, dass das Verbindungsstück im oder am Basiselement ausgebildet ist. Für einen ausreichenden Halt ist es an sich schon genügend, wenn insgesamt nur zwei oder drei Verbindungsstege vorgesehen sind. Für einen besonders guten Halt und für wenig Verzug ist allerdings bevorzugt vorgesehen, dass jedes Glied der Dentalkonstruktion über jeweils einen Verbindungssteg mit dem Basiselement verbunden ist. Die Aufhängung hat hier den besonderen Vorteil, dass dann die beim Sintern weich werdenden Verbindungselemente größtenteils auf Zug beansprucht werden. Dadurch kommt es höchstens zu einer leichten Streckung bzw. Längung der Verbindungselemente, es kommt aber zu keiner Verbiegung oder Verkippung. Besonders effizient lässt sich das Dentalwerkstück herstellen, wenn die Dentalkonstruktion, die Verbindungsstege und das Basiselement einstückig ausgebildet sind und das bearbeitete Dentalwerkstück bilden. Für einen geringen Materialverbrauch ist zudem vorgesehen, dass das Basiselement plattenförmig ausgebildet ist. Die Verbindungsstege werden nach dem Sintern, vorzugsweise durch eine Trennvorrichtung, von der Dentalkonstruktion getrennt.

Die prinzipielle Ausbildung des Verbindungsstücks ist an sich beliebig, solange mit diesem oder über dieses Verbindungsstück das Aufhängen des Dentalwerkstücks an der Haltevorrichtung ermöglicht wird und dieses Verbindungsstück gesondert von der Dentalkonstruktion ausgebildet ist. Für eine einfache und unkomplizierte Ausbildung ist allerdings bevorzugt vorgesehen, dass das Verbindungsstück als Ausnehmung oder als Vorsprung im Basiselement ausgebildet ist. Prinzipiell ist es hier schon möglich, dass zwei oder mehrere solcher Verbindungsstücke vorgesehen sind. Bevorzugt ist für eine einfache und schnelle Herstellung allerdings vorgesehen, dass im oder am Basiselement nur ein Verbindungsstück ausgebildet ist. Besonders um hier ein sicheres und genaues Aufhängen zu ermöglichen, ist bevorzugt vorgesehen, dass das Verbindungsstück auf einer Mittelachse des Dentalwerkstücks liegt. Diese Mittelachse entspricht der Links-Rechts-Trennebene des menschlichen Körpers und somit auch des menschlichen Gebisses. Durch die Aufhängung an dieser Mittelachse ist auch ein Aufhängen im Schwerpunkt garantiert. Besonders bevorzugt ist das Verbindungsstück möglichst im Zentrum der zirkulären Dentalkonstruktion angeordnet bzw. ausgebildet.

Für die Durchführung der Aufhängung ist bevorzugt ein Aufhängelement vorgesehen, wobei das Dentalwerkstück über das Aufhängelement, vorzugsweise über ein einziges Aufhängelement, an der Haltevorrichtung aufgehängt ist. Zum Beispiel soll nicht ausgeschlossen werden, dass das Aufhängelement als Seil, als Klebemittel oder als Einschnappelement ausgebildet ist. Bezüglich dieses Aufhängelements gibt es aber die drei folgenden bevorzugten und unterschiedlichen Ausführungsvarianten.

Gemäß einer solchen Ausführungsvariante kann das Aufhängelement als Klemmvorrichtung ausgebildet sein. Dabei weist diese Klemmvorrichtung, vorzugsweise zwei, Klemmbacken auf, welche in Klemmstellung an gegenüberliegenden Seiten am Basiselement anliegen. Bei dieser Ausführungsvariante wird das Verbindungsstück durch die, die Klemmbacken kontaktierenden Oberflächen des Basiselements gebildet. Es ist also keine Ausnehmung oder kein Vorsprung am Basiselement als Verbindungsstück notwendig.

Gemäß einer anderen Ausführungsvariante ist vorgesehen, dass das Aufhängelement durch das als Vorsprung ausgebildete Verbindungsstück gebildet ist und mit einer in der Haltevorrichtung ausgebildeten Ausnehmung, welche ein Verbindungsgegenstück bildet, lösbar verbunden ist. Bei dieser Variante muss also bei der Herstellung bzw. beim Fräsen des Dentalwerkstücks dieser Vorsprung im Bereich des Basiselements stehen bleiben, über welchen dann das Dentalwerkstück direkt in der Haltevorrichtung aufgehängt wird.

Gemäß einer weiteren alternativen Ausführungsvariante ist aber auch die umgekehrte Konstruktion möglich, wonach das Aufhängelement als vom Dentalwerkstück separates Verbindungsgegenstück ausgebildet ist, wobei das Aufhängelement über das als Ausnehmung im Dentalwerkstück ausgebildete Verbindungsstück mit dem Dentalwerkstück lösbar verbunden ist. Somit ragt das Aufhängelement (=Verbindungsgegenstück) in die Ausnehmung (Verbindungsstück) hinein, wodurch das Dentalwerkstück am Verbindungsgegenstück hängend gehalten ist. Bei dieser Variante gibt es wiederum zwei unterschiedliche Ausführungsformen.

Gemäß einer ersten Ausführungsform ist zur letztgenannten Ausführungsvariante vorgesehen, dass das Aufhängelement als an der Haltevorrichtung ausgebildeter, mit der Haltevorrichtung einstückiger Vorsprung ausgebildet ist. Somit sind wieder insgesamt nur zwei Teile - nämlich das Dentalwerkstück und die Haltevorrichtung - vorgesehen, wobei eben das Aufhängelement einstückig mit der Haltevorrichtung ausgebildet ist.

Gemäß einer zweiten, bevorzugten Ausführungsform zur letztgenannten Ausführungsvariante ist allerdings vorgesehen, dass das Aufhängelement von der Haltevorrichtung gesondert ausgebildet und an der Haltevorrichtung bewegbar gelagert ist. Dieses Aufhängelement kann dabei aus jedem beliebigen Material (oder aus einer Materialmischung) bestehen, welches den Temperaturbeanspruchungen beim Sintern standhält. Bevorzugt werden Keramik, Metall oder Metalllegierungen verwendet. Bei dieser Ausführungsform besteht somit die gesamte Anordnung aus drei separaten Bauteilen, nämlich aus dem Dentalwerkstück, der Haltevorrichtung und dem separaten Aufhängelement. Hierbei ist bevorzugt vorgesehen, dass das Aufhängelement an einer in der Haltevorrichtung ausgebildeten, vorzugsweise horizontal ausgerichteten, Ausnehmung bewegbar gelagert ist. Für eine besonders einfache Ausbildung und sicheren Halt ist vorgesehen, dass das, vorzugsweise bolzenförmig ausgebildete, Aufhängelement (entspricht dem Verbindungsgegenstück) bei aufgehängtem Dentalwerkstück sowohl durch die Ausnehmung in der Haltevorrichtung als auch durch die, das Verbindungsstück bildende Ausnehmung im Basiselement des Dentalwerkstücks hindurchragt.

Die Ausbildung der Haltevorrichtung ist an sich auch beliebig, solange ein sicheres Aufhängen des Dentalwerkstücks gewährleistet wird. Eine einfache Konstruktion sieht allerdings vor, dass die Haltevorrichtung zwei seitliche, vorzugsweise scheibenförmig ausgebildete, Steher und einen die Steher verbindenden Träger aufweist. Besonders bevorzugt besteht die Haltevorrichtung im Wesentlichen aus Aluminiumoxidschaum.

Das Dentalwerkstück kann aus Zirkon oder anderen sinterbaren Materialien gefertigt werden. Bevorzugt besteht allerdings das Dentalwerkstück im Wesentlichen aus Dentalmetallen, wie beispielsweise einer Chrom-Kobalt-Legierung. Da bei Dentalmetallen das Risiko eines Verzugs noch größer als bei Zirkon oder anderen sinterbaren Materialien ist, ist die vorliegende Erfindung besonders gut für solche Dentalmetallarbeiten geeignet.

Zusätzlich zum Dentalwerkstück und der Haltevorrichtung weist die Anordnung einen Sinterofen auf, wobei die Haltevorrichtung samt daran aufgehängtem Dentalwerkstück beim Sintern in einen Ofenraum des Sinterofens angeordnet ist. Um beim Sintern eine gute Stabilität und die richtige Positionierung des Dentalwerkstücks zu gewährleisten, ist bevorzugt ein verschließbares, rohrförmiges Gehäuse vorgesehen, wobei die Haltevorrichtung samt daran aufgehängtem Dentalwerkstück beim Sintern im Ofenraum des Sinterofens im Gehäuse angeordnet ist. Besonders bevorzugt ist vorgesehen, dass der Sinterofen einen eine Tür des Sinterofen bildenden Aufsatz aufweist, wobei das Gehäuse durch den Aufsatz hindurch zumindest bereichsweise in den Ofenraum hineinragt. Für das Vermeiden von unerwünschten Oxidationen beim Sintern ist bevorzugt eine Vorrichtung zum Erzeugen eines Vakuums im Gehäuse oder zur Einleitung von Schutzgas in das Gehäuse vorgesehen.

Die Aufgabe der vorliegenden Erfindung wird auch durch ein Verfahren nach Anspruch 15 gelöst. Bei diesem Verfahren zum Sintern eines Dentalwerkstücks mit einem Dentalwerkstück, einer Haltevorrichtung für das Dentalwerkstück und einem Sinterofen sind demnach die erfindungsgemäßen Schritte Bearbeiten des Dentalwerkstücks, sodass das Dentalwerkstück eine Dentalkonstruktion mit zumindest acht, miteinander verbundenen Gliedern aufweist, (wobei jedes Glied zumindest bereichsweise einem Zahnersatz eines einzelnen menschlichen Zahnes entspricht), Aufhängen des Dentalwerkstück an der Haltevorrichtung durch Verbinden des Dentalwerkstücks mit der Haltevorrichtung über ein im oder am Dentalwerkstück ausgebildetes, von der Dentalkonstruktion gesondertes Verbindungsstück und Sintern, vorzugsweise sauerstofffreies Sintern, des an der Haltevorrichtung aufgehängten Dentalwerkstücks vorgesehen.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen:
- Fig. 1: eine perspektivische Darstellung der Anordnung,
- Fig. 2: eine Seitenansicht der Anordnung,
- Fig. 3: einen Ofenaufsatz beim Einbringen der Anordnung,
- Fig. 3a: ein Detail aus Fig. 3,
- Fig. 4: eine Frontansicht des Aufsatzes,
- Fig. 5: einen Schnitt durch den Aufsatz,
- Fig. 5a: ein Detail aus Fig. 5
- Fig. 6a - 6c: unterschiedliche Ansichten einer weiteren Aufhängvariante,
- Fig. 7a - 7c: unterschiedliche Ansichten einer Aufhängvariante für ein relativ kleines Dentalwerkstück,
- Fig. 8a - 8d: unterschiedliche Ansichten und ein Schnitt einer Aufhängvariante mit einer Klemmvorrichtung,
- Fig. 9a & 9b: unterschiedliche Ansichten eines Dentalwerkstücks mit einem Steg und
- Fig. 10a & 10b: unterschiedliche Ansichten des Stegs bei entferntem Basiselement.

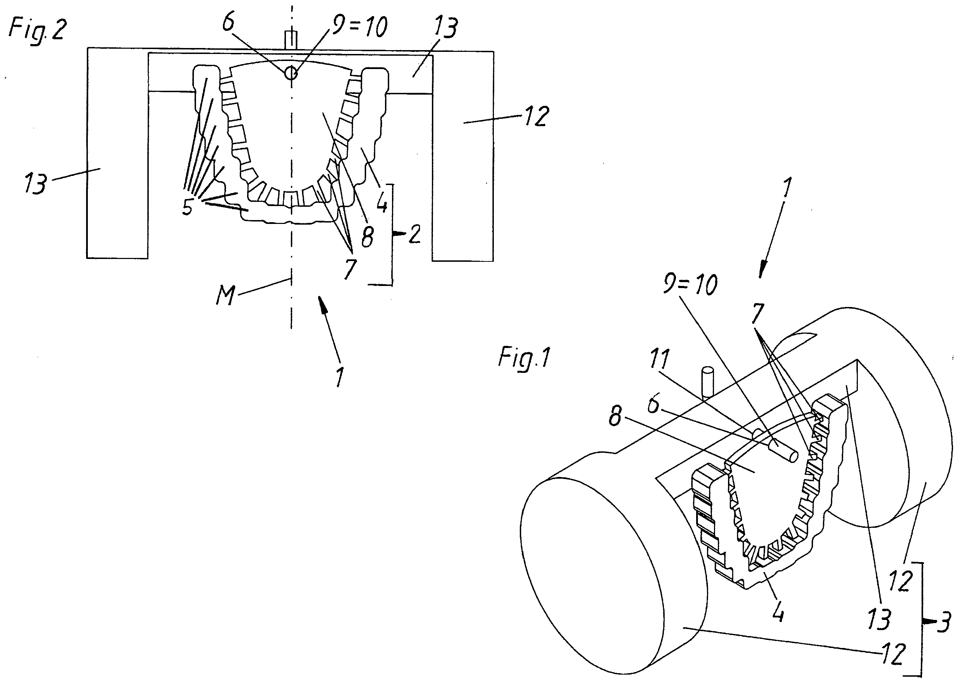

In den Fig. 1 und 2 ist eine Anordnung 1 dargestellt, welche einerseits die Haltevorrichtung 3 und andererseits das bearbeitete Dentalwerkstück 2 aufweist. Die Haltevorrichtung 3 umfasst dabei zwei seitliche, scheibenförmige Steher 12, die über einen Träger 13 verbunden sind. Diese Haltevorrichtung 3 ist einstückig ausgebildet und besteht aus einem harten Aluminiumoxidschaum. Demgegenüber kann das Dentalwerkstück 2 z. B. aus Zirkon oder aus Dentalmetall bestehen. Dieses in einer Bearbeituhgs- bzw. Fräsvorrichtung bearbeitete Dentalwerkstück 2 weist eine mindestens fünfgliedrige, vorzugsweise mindestens achtgliedrige, Dentalkonstruktion 4 auf. Die einzelnen Glieder 5 dieser zirkulären Dentalkonstruktion 4 sind jeweils über Verbindungsstege 7 mit einem plattenförmigen Basiselement 8 verbunden. In der Mittelachse M dieses Dentalwerkstücks 2 ist im Basiselement 8 das Verbindungsstück 6 in Form einer Ausnehmung ausgebildet. Über das bolzenförmig ausgebildete Aufhängelement 9 - welches das Verbindungsgegenstück 10 zum Verbindungsstück 6 bildet - ist das Dentalwerkstück 2 an der Haltevorrichtung 3 aufgehängt, wobei das Aufhängelement 9 in einer Ausnehmung 11 an der Haltevorrichtung 3 lösbar verbunden ist. Das Aufhängelement 9 verbindet somit das Dentalwerkstück 2 mit der Haltevorrichtung 3. In diesem Fall besteht somit die Anordnung 1 aus den drei Komponenten Dentalwerkstück 2, Haltevorrichtung 3 und Aufhängelement 9.

Im Gegensatz zum bevorzugten Ausführungsbeispiel gemäß den Fig. 1 und 2 kann die Aufhängung auch andersartig ausgestaltet sein. So kann beispielsweise das Verbindungsstück 6 als Vorsprung ausgebildet sein und direkt in die Ausnehmung 11 - die dann das Verbindungsgegenstück 10 bildet - in der Haltevorrichtung 3 eingreifen. Auch ist es möglich, dass das Aufhängelement 9 einstückig mit der Haltevorrichtung 3 ausgebildet ist und somit von dieser vorsteht und an diesem Aufhängelement 9 dann das Dentalwerkstück 2 über das als Ausnehmung ausgebildete Verbindungsstück 6 aufgehängt ist.

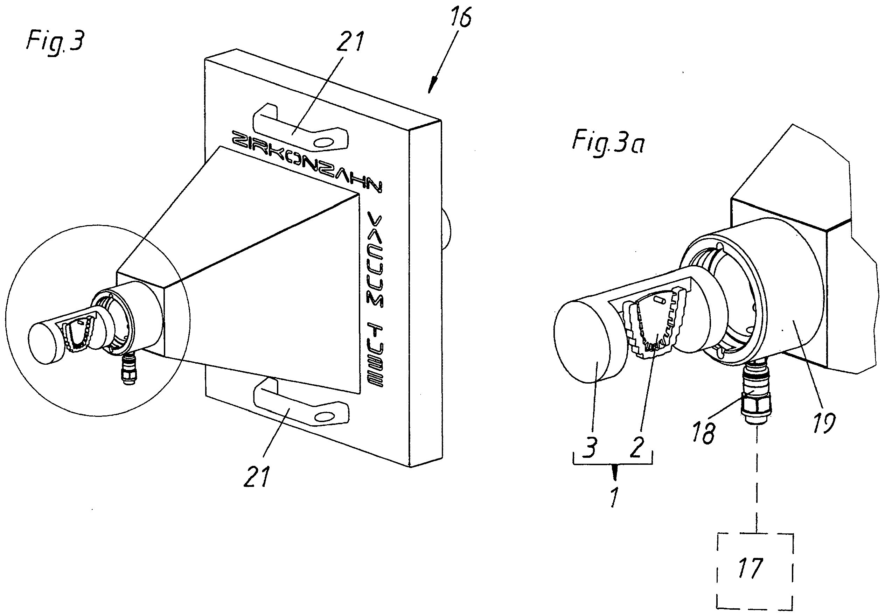

Die Fig. 3 zeigt einen Aufsatz 16, der eine Tür eines Sinterofens 14 bildet. An diesem Aufsatz 16 sind Türgriffe 21 montiert. In Fig. 3a ist der vordere Teil dieses Aufsatzes 16 mit der Einführöffnung 19 erkennbar. Von dieser Einführöffnung 19 führt auch der Anschluss 18 weg, über den eine Verbindung mit der nur strichliert angedeuteten Vorrichtung 17 zum Erzeugen eines Vakuums oder zur Einleitung von Schutzgas verbunden ist.

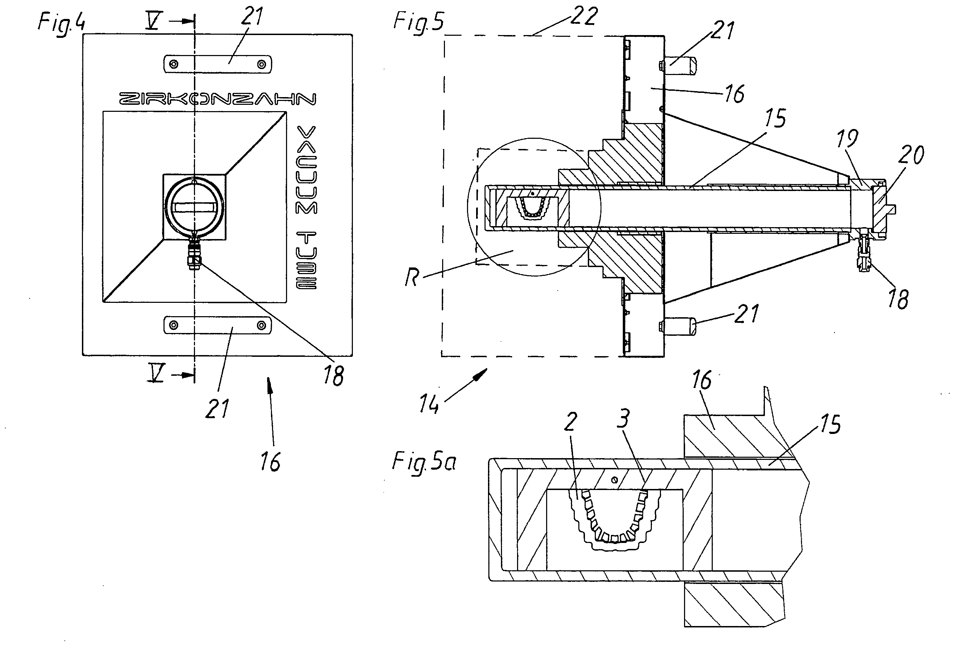

Fig. 4 zeigt eine Frontansicht des Aufsatzes 16, wobei auch hier wiederum der Anschluss 18 und die Türgriffe 21 erkennbar sind.

Fig. 5 zeigt einen Schnitt durch den Aufsatz 16, wobei auch das im Aufsatz 16 lösbar gehaltene, rohrförmige Gehäuse 15 dargestellt ist. Dieses Gehäuse 15 bildet ein Vakuumsinterrohr. Das Gehäuse 15 ist durch den Verschlussdeckel 20 verschließbar, wodurch nach Einleitung eines Schutzgases bzw. nach Erzeugung eines Vakuums über den Anschluss 18 das im Gehäuse 15 angeordnete Dentalwerkstück 2 im Sinterofen 14 gesintert wird. Das Ofengehäuse 22 des Sinterofens 14 ist in Fig. 5 nur durch Strichlierung angedeutet. Es ist aber gut ersichtlich, dass das an der Haltevorrichtung 3 aufgehängte Dentalwerkstück im Ofenraum R (Brennraum) dieses Sinterofens 14 angeordnet ist. In Fig. 5a ist auch gut ersichtlich, dass die Außenkontur der Haltevorrichtung 3 bzw. insbesondere der scheibenförmigen Steher 12 an die Innenkontur des rohrförmigen Gehäuses 15 angepasst ist, wodurch ein sicherer Halt gegeben ist.

Durch das Aufhängen des mindestens fünfgliedrigen Dentalwerkstücks 2 an einer Haltevorrichtung 3 beim Sintern in einem Sinterofen 14 ist garantiert, dass es zu keinen ungewünschten Verzug kommt. Natürlich kann das Dentalwerkstück 2 auch direkt im Ofenraum R - also ohne Vakuumsinterrohr bzw. Gehäuse 15 - gesintert werden. Dann ist der Sinterofen 14 anstatt mit dem Aufsatz 16 mit einer normalen Türe verschlossen. Bei einem derartigen Einsatz kann die Haltevorrichtung 3 natürlich auch eine andere Form mit einer ebenen Stehfläche aufweisen.

In den Fig. 6a, 6b und 6c sind drei unterschiedliche Ansichten einer weiteren Ausführungsvariante einer Anordnung 1 dargestellt. In diesem Fall ist die Ausnehmung 11 in der Haltevorrichtung 3 als eine längliche Vertiefung an der Oberseite des Trägers 13 der Haltevorrichtung 3 ausgebildet. In dieser Vertiefung liegt das Aufhängelement 9 auf. Das Verbindungsstück 6 ist als Ausnehmung im obersten Bereich des plattenförmigen Basiselements 8 ausgebildet. Über diese Ausnehmung hält das Aufhängelement 9 das Dentalwerkstück 2 an der Haltevorrichtung 3. Die Steher 12 sind bei dieser Ausführung ringförmig und mit einem abgeflachten Stehbereich ausgebildet.

Die Fig. 7a, 7b und 7c zeigen unterschiedliche Ansichten einer Anordnung 1 mit einer relativ kleinen, in diesem Falle fünfgliedrigen Dentalkonstruktion 4, welche über Verbindungsstege 7 mit einem horizontal ausgerichteten Basiselement 8 verbunden ist. Das Aufhängelement 9 weist Haltevorsprünge 23 auf, auf welchen das Basiselement 8 aufliegt. Diese Aufliegeflächen des Basiselements 8 bilden in diesem Fall das Verbindungsstück 6. Gleich wie bei der vorherigen Variante ist auch hier wieder das Aufhängelement 9 in einer als längliche Vertiefung ausgebildeten Ausnehmung 11 an der Haltevorrichtung 3 aufgehängt. Es können auch mehrere solcher relativ kleiner Dentalwerkstücke 2 über ein oder mehrere Aufhängelemente 9 an einer Haltevorrichtung 3 aufgehängt werden.

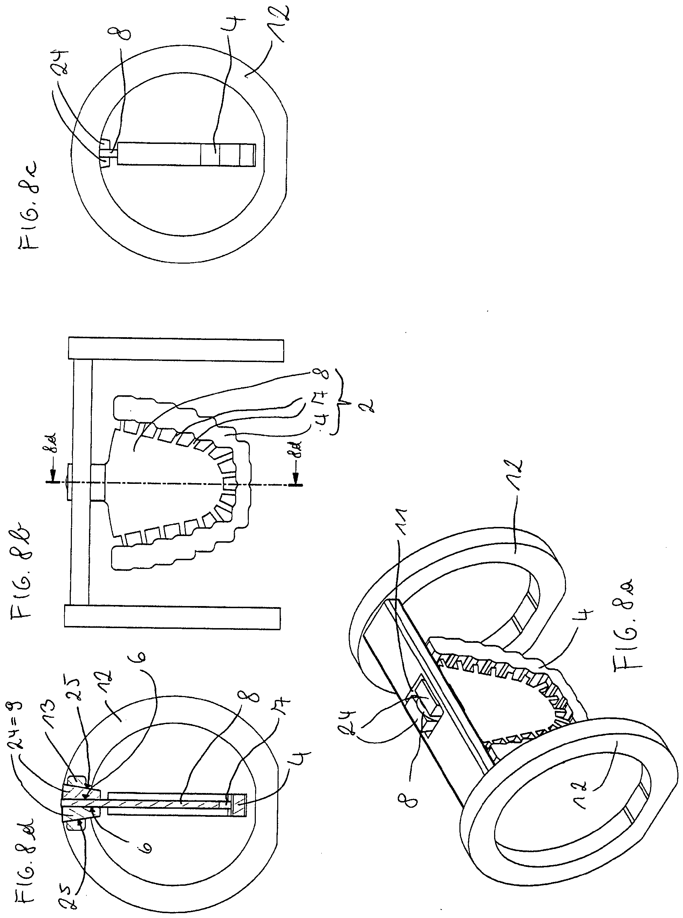

In den Fig. 8a, 8b, 8c und 8d sind drei unterschiedliche Ansichten und ein Schnitt durch noch eine weitere Ausführungsvariante einer Anordnung 1 abgebildet. Gemäß dieser Variante wird das Aufhängelement 9 durch eine Klemmvorrichtung gebildet. Diese Klemmvorrichtung weist zwei Klemmbacken 24 auf, welche von oben in eine mit schrägen Seitenflächen versehene Ausnehmung 11 im Träger 13 eingesetzt wird. Dabei kommen die ebenfalls abgeschrägten Keilflächen 25 der Klemmbacken 24 in Kontakt mit den schrägen Seitenflächen der Ausnehmung 11. Dadurch werden die Klemmbacken 24 zueinander bewegt, wodurch das zwischen den Klemmbacken 24 befindliche Basiselement 8 des Dentalwerkstücks 2 in der Klemmvorrichtung eingeklemmt und dadurch an der Haltevorrichtung 3 aufgehängt wird. Das Verbindungsstück 6 wird in diesem Fall durch die an den Innenseiten der Klemmbacken 24 anliegenden Oberflächenbereiche des Basiselements 8 gebildet.

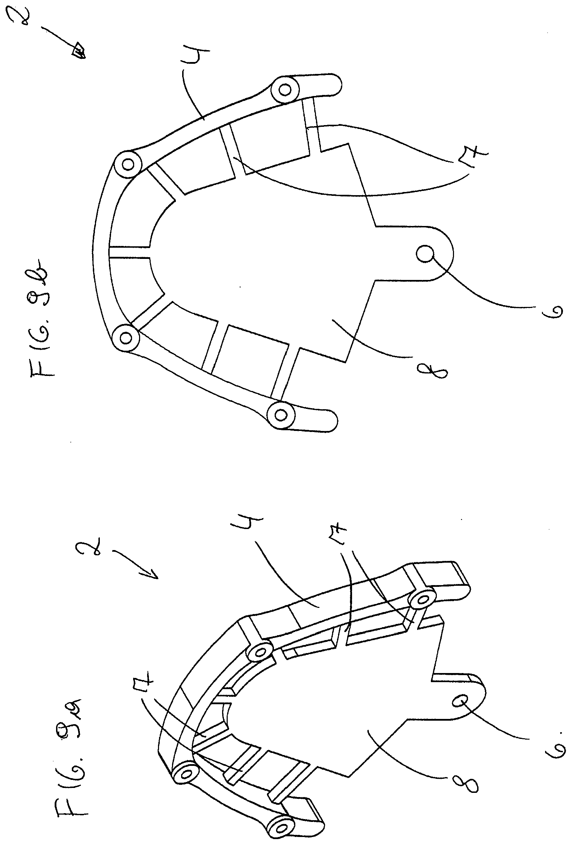

Die Fig. 9a und 9b zeigen unterschiedliche Ansichten eines Dentalwerkstücks 2, bei welchem die Dentalkonstruktion 4 nicht als im Wesentlichen vollständiger Zahnersatz (wie bei allen vorherigen schematischen Figuren) sondern als Steg ausgebildet ist. Dies kann zum Beispiel über eine Klippverbindung oder andere, dem Zahntechniker bekannte Methoden erfolgen. Auch ein solches Dentalwerkstück 2 kann wie beschrieben an einer Haltevorrichtung 3 aufgehängt werden.

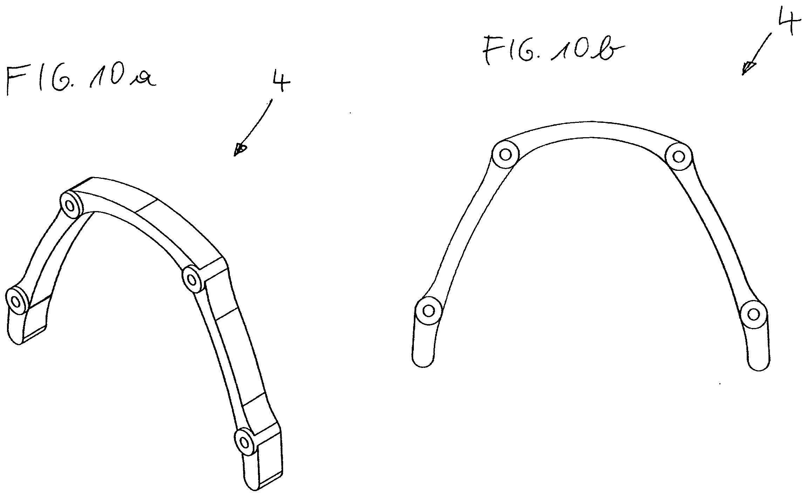

Auf einen solchen Steg - welcher in den Fig. 10 und 10b ohne Verbindungsstege 7 und Basiselement 8 dargestellt ist - wird dann in späterer Folge eine entsprechender Zahnersatz aufgesetzt bzw. aufgebaut.

### Bezugszeichenliste:

- 1: Anordnung
- 2: Dentalwerkstück
- 3: Haltevorrichtung
- 4: Dentalkonstruktion
- 5: Glieder
- 6: Verbindungsstück
- 7: Verbindungsstege
- 8: Basiselement
- 9: Aufhängelement
- 10: Verbindungsgegenstück
- 11: Ausnehmung in Haltevorrichtung
- 12: Steher
- 13: Träger
- 14: Sinterofen
- 15: Gehäuse
- 16: Aufsatz
- 17: Vorrichtung (für Vakuumerzeugung oder Schutzgaseinleitung)
- 18: Anschluss
- 19: Einführöffnung
- 20: Verschlussdeckel
- 21: Türgriffe
- 22: Ofengehäuse
- 23: Haltevorsprünge
- 24: Klemmbacken
- 25: abgeschrägte Keilflächen
- M: Mittelachse
- R: Ofenraum

## Patentansprüche

1. Anordnung (1) zum Sintern eines Dentalwerkstücks (2), mit einem bearbeiteten Dentalwerkstück (2) und einer Haltevorrichtung (3) für das Dentalwerkstück (2), wobei
- das Dentalwerkstück (2) eine Dentalkonstruktion (4) mit zumindest fünf, vorzugsweise mit zumindest acht, miteinander verbundenen Gliedern (5) aufweist,
- das Dentalwerkstück (2) an der Haltevorrichtung (3) aufgehängt ist und
- das Dentalwerkstück (2) über ein im oder am Dentalwerkstück (2) ausgebildetes, von der Dentalkonstruktion (4) gesondertes Verbindungsstück (6) mit der Haltevorrichtung (3) verbunden bzw. verbindbar ist.

2. Anordnung nach Anspruch 1, wobei die Dentalkonstruktion (4) als zumindest bereichsweise, vorzugsweise vollständig, zirkulär geformte Brückenkonstruktion oder als zumindest bereichsweise, vorzugsweise vollständig, zirkulär geformter Steg ausgebildet ist.

3. Anordnung nach Anspruch 1 oder 2, wobei das Dentalwerkstück (2) Verbindungsstege (7) und ein, vorzugsweise plattenförmiges, Basiselement (8) aufweist, wobei die Dentalkonstruktion (4) über die Verbindungsstege (7) mit dem Basiselement (8) verbunden ist.

4. Anordnung nach Anspruch 3, wobei das Verbindungsstück (6) im oder am Basiselement (8) ausgebildet ist.

5. Anordnung nach Anspruch 3 oder 4, wobei die Dentalkonstruktion (4), die Verbindungsstege (7) und das Basiselement (8) einstückig ausgebildet sind und das bearbeitete Dentalwerkstück (2) bilden.

6. Anordnung nach einem der Ansprüche 3 bis 5, wobei das Verbindungsstück (6) als Ausnehmung oder als Vorsprung im Basiselement (8) ausgebildet ist.

7. Anordnung nach einem der Ansprüche 1 bis 6, mit einem Aufhängelement (9), wobei das Dentalwerkstück (2) über das Aufhängelement (9), vorzugsweise über ein einziges Aufhängelement (9), an der Haltevorrichtung (3) aufgehängt ist.

8. Anordnung nach Anspruch 7, wobei das Aufhängelement (9) durch das als Vorsprung ausgebildete Verbindungsstück (6) gebildet ist und mit einer in der Haltevorrichtung (3) ausgebildeten Ausnehmung, welche ein Verbindungsgegenstück (10) bildet, lösbar verbunden ist.

9. Anordnung nach Anspruch 7, wobei das Aufhängelement (9) als vom Dentalwerkstück (2) separates Verbindungsgegenstück (10) ausgebildet ist, wobei das Aufhängelement (9) über das als Ausnehmung im Dentalwerkstück (2) ausgebildete Verbindungsstück (6) mit dem Dentalwerkstück (2) lösbar verbunden ist.

10. Anordnung nach Anspruch 9, wobei das Aufhängelement (9) als an der Haltevorrichtung (3) ausgebildeter, mit der Haltevorrichtung (3) einstückiger Vorsprung ausgebildet ist.

11. Anordnung nach Anspruch 9 , wobei das Aufhängelement (9) von der Haltevorrichtung (3) gesondert ausgebildet und an der Haltevorrichtung (3) bewegbar gelagert ist.

12. Anordnung nach Anspruch 11, wobei das Aufhängelement (9) an einer in der Haltevorrichtung (3) ausgebildeten, vorzugsweise horizontal ausgerichteten, Ausnehmung (11) bewegbar gelagert ist.

13. Anordnung nach Anspruch 11 oder 12, wobei das, vorzugsweise bolzenförmig ausgebildete, Aufhängelement (9) bei aufgehängtem Dentalwerkstück (2) sowohl durch die Ausnehmung (11) in der Haltevorrichtung (3) als auch durch die, das Verbindungstück (6) bildende Ausnehmung im Basiselement (8) des Dentalwerkstücks (2) hindurchragt.

14. Anordnung nach Anspruch 7, wobei das Aufhängelement (9) als Klemmvorrichtung ausgebildet ist, welche in einer Klemmstellung über Klemmbacken (24) am Basiselement (8) anliegt.

15. Verfahren zum Sintern eines Dentalwerkstücks (2), mit einem Dentalwerkstück (2), einer Haltevorrichtung (3) für das Dentalwerkstück (2) und einem Sinterofen (14), mit den Schritten:
- Bearbeiten des Dentalwerkstücks (2), sodass das Dentalwerkstück (2) eine Dentalkonstruktion (4) mit zumindest fünf, vorzugsweise mit zumindest acht, miteinander verbundenen Gliedern (5) aufweist,
- Aufhängen des Dentalwerkstück (2) an der Haltevorrichtung (3) durch Verbinden des Dentalwerkstücks (2) mit der Haltevorrichtung (3) über ein im oder am Dentalwerkstück (2) ausgebildetes, von der Dentalkonstruktion (4) gesondertes Verbindungsstück (6) und
- Sintern, vorzugsweise sauerstofffreies Sintern, des an der Haltevorrichtung (3) aufgehängten Dentalwerkstücks (2).

16. Verfahren nach Anspruch 15, wobei das Dentalwerkstück (2) Verbindungsstege (7) und eine Basiselement (8) aufweist und die Dentalkonstruktion (4) über die Verbindungsstege (7) mit dem Basiselement (8) verbunden ist, wobei das Verbindungsstück (6) im oder am Basiselement (8) ausgebildet ist.

## Claims

1. An arrangement (1) for sintering a dental workpiece (2) with a processed dental workpiece (2) and a holding device (3) for the dental workpiece (2), wherein
- the dental workpiece (2) comprises a dental construction (4) with at least five, preferably with at least eight, interconnected members (5),
- the dental workpiece (2) is suspended on the holding device (3), and
- the dental workpiece (2) is or can be connected to the holding device (3) by way of a connecting portion (6) which is provided in or on the dental workpiece (2) and is separate from the dental construction (4).

2. An arrangement according to claim 1 wherein the dental construction (4) is in the form of an at least region-wise, preferably completely, circularly shaped bridge construction or in the form of an at least region-wise, preferably completely, circularly shaped leg.

3. An arrangement according to claim 1 or claim 2 wherein the dental workpiece (2) has connecting arms (7) and a base element (8), wherein the dental construction (4) is connected to the base element (8) by way of the connecting arms (7).

4. An arrangement according to claim 3 wherein the connecting portion (6) is provided in or on the base element (8).

5. An arrangement according to claim 3 or claim 4 wherein the dental construction (4), the connecting arms (7) and the base element (8) are in one piece and form the processed dental workpiece (2).

6. An arrangement according to one of claims 1 to 5 wherein the connecting portion (6) is in the form of an opening or a projection in the base element (8).

7. An arrangement according to one of claims 1 to 6 having a suspension element (9), wherein the dental workpiece (2) is suspended on the holding device (3) by way of the suspension element (9), preferably by way of a single suspension element (9).

8. An arrangement according to claim 7 wherein the suspension element (9) is formed by the connecting portion (6) in the form of a projection and is releasably connected to an opening which is provided in the holding device (3) and which forms a connecting counterpart portion (10).

9. An arrangement according to claim 7 wherein the suspension element (9) is in the form of a connecting counterpart portion (10) separate from the dental workpiece (2), wherein the suspension element (9) is releasably connected to the dental workpiece (2) by way of the connecting portion (6) which is in the form of an opening in the dental workpiece (2).

10. An arrangement according to claim 9 wherein the suspension element (9) is in the form of a projection which is provided on the holding device (3) and which is in one piece with the holding device (3).

11. An arrangement according to claim 9 wherein the suspension element (9) is provided separately from the holding device (3) and is moveably mounted to the holding device (3).

12. An arrangement according to claim 11 wherein the suspension element (9) is moveably mounted on an opening (11) which is provided in the holding device (3) and which is preferably horizontally oriented.

13. An arrangement according to claim 11 or claim 12 wherein the suspension element (9) which is preferably of a pin-shaped configuration, with the dental workpiece (2) suspended thereon, projects both through the opening (11) in the holding device (3) and also through the opening, forming the connecting portion (6), in the base element (8) of the dental workpiece (2).

14. An arrangement according to claim 7 wherein the suspension element (9) is in the form of a clamping device which in a clamping position bears against the base element (8) by way of clamping jaws (24).

15. A method of sintering a dental workpiece (2) with a dental workpiece (2), a holding device (3) for the dental workpiece (2) and a sintering furnace (14), comprising the steps:
- processing the dental workpiece (2) so that the dental workpiece (2) has a dental construction (4) with at least five, preferably with at least eight, interconnected members (5),
- suspending the dental workpiece (2) on the holding device (3) by connecting the dental workpiece (2) to the holding device (3) by way of a connecting portion (6) which is provided in or on the dental workpiece (2) and is separate from the dental construction (4), and
- sintering, preferably oxygen-free sintering, the dental workpiece (2) suspended on the holding device (3).

16. A method according to claim 15 wherein the dental workpiece (2) has connecting arms (7) and a base element (8) and the dental construction (4) is connected to the base element (8) by way of the connecting arms (7), the connecting portion (6) being provided in or on the base element (8).

## Revendications

1. Disposition (1) destinée au frittage d'une pièce dentaire (2), avec une pièce dentaire (2) usinée et un dispositif de maintien (3) pour la pièce dentaire (2), selon laquelle :
- la pièce dentaire (2) présente une structure dentaire (4) avec au moins cinq, de préférence au moins huit membres (5) reliés les uns aux autres ;
- la pièce dentaire (2) est suspendue au dispositif de maintien (3) ; et
- la pièce dentaire (2) est reliée ou peut être reliée au dispositif de maintien (3) par l'intermédiaire d'une pièce de jonction (6), laquelle est distincte de la structure dentaire (4) et laquelle est placée dans ou sur la pièce dentaire (2).

2. Disposition selon la revendication 1, selon laquelle la structure dentaire (4) est formée sous la forme d'une structure en pont moulée de manière circulaire, au moins par zones, de préférence complètement, ou sous la forme d'une traverse moulée de manière circulaire, au moins par zones, de préférence complètement.

3. Disposition selon la revendication 1 ou 2, selon laquelle la pièce dentaire (2) présente des traverses de jonction (7) et un élément de base (8), de préférence en forme de plaque, selon laquelle la structure dentaire (4) est reliée à l'élément de base (8) par l'intermédiaire des traverses de jonction (7).

4. Disposition selon la revendication 3, selon laquelle la pièce de jonction (6) est placée dans ou sur l'élément de base (8).

5. Disposition selon la revendication 3 ou 4, selon laquelle la structure dentaire (4), les traverses de jonction (7) et l'élément de base (8) sont formés d'une seule pièce et forment la pièce dentaire (2) usinée.

6. Disposition selon l'une des revendications 3 à 5, selon laquelle la pièce de jonction (6) est conçue sous la forme d'une cavité ou sous la forme d'une saillie dans l'élément de base (8).

7. Disposition selon l'une des revendications 1 à 6, avec un élément de suspension (9), selon laquelle la pièce dentaire (2) est suspendue au dispositif de maintien (3) par l'intermédiaire de l'élément (9), de préférence par l'intermédiaire d'un seul élément (9).

8. Disposition selon la revendication 7, selon laquelle l'élément (9) est formé par la pièce de jonction (6), laquelle est conçue sous la forme d'une saillie, et est relié de manière amovible à une cavité conçue dans le dispositif de maintien (3), laquelle cavité forme une pièce de jonction opposée (10).

9. Disposition selon la revendication 7, selon laquelle l'élément (9) est formé sous la forme d'une pièce de jonction opposée (10), séparée de la pièce dentaire (2), selon laquelle l'élément (9) est relié de manière amovible à la pièce dentaire (2) par l'intermédiaire de la pièce de jonction (6), laquelle est formée sous la forme d'une cavité dans la pièce dentaire (2).

10. Disposition selon la revendication 9, selon laquelle l'élément (9) est conçu sous la forme d'une saillie d'une seule pièce avec le dispositif de maintien (3), laquelle saillie est placée sur le dispositif de maintien (3).

11. Disposition selon la revendication 9, selon laquelle l'élément (9) est formé de manière distincte du dispositif de maintien (3) et est logé de manière mobile sur le dispositif de maintien (3).

12. Disposition selon la revendication 11, selon laquelle l'élément (9) est logé de manière mobile sur une cavité (11), laquelle est formée dans le dispositif de maintien (3), et laquelle s'étend de préférence à l'horizontale.

13. Disposition selon la revendication 11 ou 12, selon laquelle, quand la pièce dentaire (2) est suspendue, l'élément (9), lequel est, de préférence, conçu en forme de boulon, pénètre aussi bien à travers la cavité (11) dans le dispositif de maintien (3) qu'à travers la cavité formant la pièce de jonction (6), dans l'élément de base (8) de la pièce dentaire (2).

14. Disposition selon la revendication 7, selon laquelle l'élément (9) est conçu sous la forme d'un dispositif de serrage, lequel s'appuie sur l'élément de base (8) dans une position de serrage, par des mâchoires de serrage (24).

15. Procédé destiné au frittage d'une pièce dentaire (2), avec une pièce dentaire (2), un dispositif de maintien (3) pour la pièce dentaire (2) et un four de frittage (14), lequel procédé comprend les phases suivantes :
- le traitement de la pièce dentaire (2), de telle sorte que la pièce dentaire (2) présente une structure dentaire (4) avec au moins cinq, de préférence au moins huit membres (5) reliés les uns aux autres ;
- la suspension de la pièce dentaire (2) au dispositif de maintien (3) par la jonction de la pièce dentaire (2) avec le dispositif de maintien (3) par une pièce de jonction (6), laquelle est distincte de la structure dentaire (4) et laquelle est formé dans ou sur la pièce dentaire (2) ;
- le frittage, de préférence le frittage exempt d'oxygène, de la pièce dentaire (2) suspendue au dispositif de maintien (3).

16. Procédé selon la revendication 15, selon lequel la pièce dentaire (2) présente des traverses de jonction (7) et un élément de base (8) ; et
selon lequel la structure dentaire (4) est reliée à l'élément de base (8) par l'intermédiaire des traverses de jonction (7) ;
selon lequel la pièce de jonction (6) est conçue dans ou sur l'élément de base (8).
